# EUROPÄISCHE PATENTSCHRIFT

(11) **EP 2 125 045 B1**
(45) Veröffentlichungstag und Bekanntmachung des Hinweises auf die Patenterteilung: **22.08.2012**
(21) Anmeldenummer: 07819389.3
(22) Anmeldetag: 27.10.2007
(51) Int. Cl.: A61L 9/04, A61L 9/12, A61L 9/14

(54) **VORRICHTUNG UND VERFAHREN ZUR ABGABE VON DÜFTEN**
DEVICE AND METHOD FOR RELEASING FRAGRANCES
DISPOSITIF ET PROCÉDÉ POUR DIFFUSER DES SENTEURS

(30) Priorität: 16.02.2007 DE 102007007745
(43) Veröffentlichungstag der Anmeldung: 02.12.2009
(73) Patentinhaber: Jerichow, Ulrich, 63571 Gelnhausen (DE)
(72) Erfinder: Jerichow, Ulrich, 63571 Gelnhausen (DE)
(74) Vertreter: Metten, Karl-Heinz
(86) Internationale Anmeldenummer: PCT/EP2007/009346
(87) Internationale Veröffentlichungsnummer: WO 2008/098597

(56) Entgegenhaltungen:
- EP-A- 1 468 702
- WO-A-2004/043502
- WO-A-2004/049237
- US-A1- 2002 043 568
- US-A1- 2006 144 956

## Beschreibung

Die vorliegende Erfindung betrifft eine Vorrichtung und ein Verfahren zur Abgabe von Düften, welche insbesondere für die Integration in ein System zur Erzeugung einer virtuellen Realität geeignet sind.

Vorrichtungen verschiedener Art zur Abgabe von Duftstoffen sind bereits seit langem bekannt. So wird gemäß der DE 10 2005 010 713 eine Vorrichtung zur Abgabe von Duftstoffen beschrieben, welche aus einem Gehäuse mit einem Vorratsbehälter für die Duftstofflösung, einer mit einer elektrischen Heizeinrichtung erwärmbaren Verdampfungsschale, einer elektrochemischen Gasentwicklungszelle und einem Ventilator über der Verdampfungsschale besteht.

Gemäß der DE 20 2004 016 162 ist ein Auslasskopf für eine Sprühvorrichtung bekannt, wobei der Auslasskopf einen Einlassabschnitt mit einer Öffnung und einen Auslassabschnitt, der daran angepasst ist, Sprühmaterial auszustoßen, umfasst. Weiterhin ist gemäß der DE 699 26 134 ein Verfahren und eine Vorrichtung zum Erzeugen einer Parfümzusammensetzung bekannt, die wenigstens zwei Behälter mit Sprüheinheiten aufweist, wobei die Behälter mit flüssigen Parfümprodukten befüllt werden können, die Behälter mit flüssigen Parfümprodukten durch Pipelines an Spender angeschlossen sind und die Spender und Injektoren zum Einspritzen von Mikroströmen an Mikrodüsen angeschlossen sind. Dabei sind die Mikrodüsen in gegenseitiger Nähe angeordnet und die Vorrichtung ist autonom und mobil ausgeführt, wobei die Vorrichtung ferner eine an einen Hauptspeicher angeschlossene Mikroprozessorvorrichtung zum Steuern der Parameter der Spender und der Injektoren und eine von einem Benutzer gesteuerte Schnittstelle aufweist.

Gemäß der US 4 629 604 ist ein Abspielgerät für eine Mehrfach-Aromapatrone beschrieben, welches u. a. aus einer planaren Anordnung rahmenähnlicher Einheiten besteht, wobei ein Kissen einer jeden Einheit mit einem flüssigen Duft getränkt ist. Unterhalb der Einheiten sind einzeln elektrisch beheizbare Zellen in Form von Honigwaben angeordnet, sodass gezielt ein gewünschter Duft aus einer der Einheiten freigesetzt werden kann.

Eine als Duftspender ausgebildete Vorrichtung mit mehreren, jeweils einen verschließbaren Duftaustrittskanal aufweisenden Duftstoffspeichern, in welchen Duftstoffe luftdicht eingeschlossen sind, wobei Duftstoffspeicher vorgesehen sind, welche jeweils einen anderen Duftstoff enthalten ist mit der DE 197 15 404 beschrieben. Dabei ist eine Auswahleinrichtung vorgesehen, mit welcher ein bestimmter Duftstoffspeicher festlegbar und der Duftaustrittskanal dieses Duftstoffspeichers zwecks Abgabe einer vorgebbaren Menge des in ihm vorhandenen Duftstoffs geöffnet werden kann. Es kann aber keine Überprüfung der Art des im Produktbehälter befindlichen Duftstoffes durch die Vorrichtung erfolgen.

Aus EP 1 185 310 B1 ist ein Gerät zur Abgabe von Düften synchron zu einer Signal- oder Taktgebereinheit mit einem Aromenspeicher, einer Steuereinheit zur Ansteuerung des Aromenspeichers und mit einer Austrageinheit zur Generierung und zum Austragen einer Duft- bzw. Aromenwolke aus dem Aromenspeicher bekannt. Dabei ist der Aromenspeicher vorzugsweise als Mikrochip oder als Chipkarte mit Speicherplätzen für verschiedene Duftstoffe vorgesehen. Die Aromen werden im Chip oder auf einem Träger in kleinen Mikrokammem oder Mikrotanks oder auf kleinen Speicherplätzen flüssig, als Feststoff, als Gel oder als Gas gespeichert. Den Duftstoff-Speicherplätzen ist jeweils ein von der Steuereinheit ansteuerbares Element zum thermischen und/oder elektrochemischen Austragen von Duftstoff zugeordnet. Beim Erwärmen der Duftstoffe können diese sich allerdings unter Umständen unerwünscht in ihren Eigenschaften verändern. Weiterhin besteht ein Nachteil dieses bekannten Systems in den kleinen Fassungsvermögen der Mikrokammern oder Mikrotanks.

Gemäß der EP 0 831 384 ist eine Vorrichtung und ein Verfahren zur Überwachung von olfaktorischen Reizen, wobei ein Duft abgegeben wird und Informationen zum abgegebenen Duft gespeichert werden, sodass der abgegebene Duft auf Grundlage der gespeicherten Informationen vor dem Freisetzen eines neuen Dufts im Wesentlichen wieder eliminiert werden kann. Dazu werden Eingabedaten über eine Schnittstelle und einen Kontroller, bestehend aus einem Steuerstromkreis und einem Funktionsstromkreis, an die Aromaspeicher bzw. Aromaabgabe-Einheiten weitergeleitet.

Darüber hinaus sind aus der EP 1 468 70 A, WO 2004/049237 A, WO 2004/043502 A und US-A-2006/0144956 Vorrichtungen zur Abgabe von Düften bekannt.

Bekannte Sprühvorrichtungen bzw. Duftspender der oben beschrieben Art haben den Nachteil, dass zwischen den Produktbehältern selbst, welche entsprechende Duftstoffe enthalten, und der übrigen Vorrichtung kein Informationsaustausch oder nur eine unidirektionale Datenübermittlung zum Zweck der Steuerung stattfinden kann. Mit den bekannten Dufterzeugem kann beispielsweise keine automatische Regelung des Sprühvorgangs in Abhängigkeit von der Art des im Produktbehälter befindlichen Duftstoffes und/oder in Abhängigkeit vom Füllstand des Duftvorratsbehälters erfolgen. Weiterhin besteht für den Verwender keine Möglichkeit, Informationen über den aktuell installierten Duftbehälter auszulesen.

Es ist somit die Aufgabe der vorliegenden Erfindung, die oben genannten Nachteile von Vorrichtungen der oben genannten Art zum Verteilen von Duftstoffen zu überwinden.

Die Aufgabe wird durch eine Vorrichtung und ein Verfahren zur Abgabe von Düften gelöst, wobei die Vorrichtung mindestens
- ein Gehäuse mit einer elektronischen Datenverarbeitungseinheit,
- eine in oder an dem Gehäuse befestigte Austrag-Einheit für die Abgabe von aerosoloder gasförmigen Duftstoffen und
- einen in oder an dem Gehäuse austauschbar befestigten mit Duftstoffen bestückten Produktbehälter aufweist und der Produktbehälter über eine weitere, unlösbar am Produktbehälter befestigte, Datenverarbeitungseinheit mit mindestens einem nichtflüchtigen Schreib-Lese-Speicher verfügt.

Bei einer vorteilhaften Ausgestaltung der Erfindung handelt es sich bei den Duftstoffen im Produktbehälter um eine aromatische Flüssigkeit, einen aromatischen Feststoff, ein aromatisches Gel und/oder einen mit einer aromatischen Flüssigkeit oder einem aromatischen Gel getränkten Feststoff.

Ferner enthält die am Produktbehälter befestigte Datenverarbeitungseinheit eine mit dem nichtflüchtigen Schreib-Lese-Speicher verbundene Kontrolleinheit zur Steuerung der Austrag-Einheit.

Eine derartige erfindungsgemäße Vorrichtung hat den Vorteil, dass die in dem nichtflüchtigen Schreib-Lese-Speicher gespeicherte Informationen über den im Produktbehälter befindlichen Duftstoff in eine Kontrolleinheit eingelesen werden können, wobei diese Kontrolleinheit u. a. für die Steuerung der Schreib- und Lesevorgänge in den bzw. aus dem nichtflüchtigen Schreib-Lese-Speicher verantwortlich ist. Da die Kontrolleinheit der Datenverarbeitungseinheit am Produktbehälter mit einer weiteren am Gehäuse befindlichen Datenverarbeitungseinheit verbunden ist, kann ein Datenaustausch erfolgen. Das hat den entscheidenden Vorteil, dass eine Ansteuerung der Mittel zur Dufterzeugung, beispielsweise der Austrag-Einheit, in Abhängigkeit von im nichtflüchtigen Schreib-Lese-Speicher gespeicherten Informationen mit Hilfe einer der Datenverarbeitüngseinheiten erfolgen kann. Weiterhin ergibt sich der Vorteil, dass die Daten an ein externes Computersystem weitergeleitet und von einem Anwender ausgelesen werden können. Es kann somit nicht zum versehentlichen Bestücken der Vorrichtung mit einem faschen bzw. unerwünschten Produktbehälter kommen.

Vorteilhaft ist es ferner, dass die am Produktbehälter befestigte Datenverarbeitungseinheit einen mit dem nichtflüchtigen Schreib-Lese-Speicher oder der Kontrolleinheit verbundenen Timer enthält.

Bei einer besonders vorteilhaften Ausgestaltung der erfindungsgemäßen Vorrichtung ist der nichtflüchtige Schreib-Lese-Speicher ein elektronischer Speicherbaustein vom Typ eines EPROMs, EEPROMs oder Flash-Speichers. Derartige oder ähnliche nichtflüchtige Speicher haben den Vorteil, dass auch bei Unterbrechung der Spannungsversorgung, die darin gespeicherten Informationen erhalten bleiben. So können die für den jeweiligen Produktbehälter spezifischen Daten unabhängig von einer Energieversorgung in dem am Produktbehälter unlösbar befestigten Schreib-Lese-Speicher gespeichert werden. Die Schreibfunktion ermöglicht dabei, dass auch während des Betriebs des Produktbehälters zusätzlich gespeicherte Daten, beispielsweise bei einem Wechsel der Produktbehälter, nicht wieder verloren gehen.

Ferner ist es von Vorteil, wenn der Produktbehälter mehrere voneinander abgetrennte und von der Kontrolleinheit separat ansteuerbare Kammern für die Speicherung verschiedener Duftstoffe besitzt.

Weiterhin ist es von Vorteil, wenn das Gehäuse und/oder der Produktbehälter einen akustischen und/oder visuellen Signalgeber aufweist.

Vorteilhaft ist es ferner, wenn die Austrag-Einheit für die Abgabe von aerosoloder gasförmigen Duftstoffen eine Verdunstungs-Einheit ist. Dabei kann es sich bei einer sehr einfachen Ausführung beispielsweise um einen regulierbaren luftdurchströmten Auslass am Produktbehälter handeln. Im geöffneten Zustand würde ein solcher Auslass verdunsteten bzw. verdampften Duftstoff von im Produktbehälter befindlicher aromatischer Flüssigkeit, aromatischem Feststoff, aromatischem Gel und/oder mit einer aromatischen Flüssigkeit oder einem aromatischen Gel getränktern Feststoff austragen. Es ist dabei somit keine nachteilige Erwärmung erforderlich, da lediglich kalte, raumtemperierte oder körperwarme Luft über die aromatische Flüssigkeit, den aromatischen Feststoff, das aromatische Gel und/oder den mit einer aromatischen Flüssigkeit oder einem aromatischen Gel getränkten Feststoff hinwegströmt. Dabei wird das Prinzip der kalten Diffusion ausgenutzt. Ein weiterer Vorteil ergibt sich dabei aus dem Umstand, dass sich bei einer solchen Ausgestaltung der Produktbehälter prinzipiell beliebig groß gestalten lässt. Folglich können durch die somit großen Mengen an aromatischer Flüssigkeit, aromatischem Feststoff, aromatischem Gel und/oder mit einer aromatischen Flüssigkeit oder einem aromatischen Gel getränktem Feststoff im Produktbehälter sehr lange Laufzeiten erreicht werden, ohne den Produktbehälter austauschen zu müssen.

Alternativ kann die Austrag-Einheit im Sinne dieser Erfindung auch eine übliche Sprühvorrichtung für die Erzeugung von Aerosolen sein. Weiterhin kann die Austrag-Einheit auch in Form von speziellen Düsen zum Ausstoßen einer Flüssigkeit ausgestaltet sein. Dabei kann der Austrag aus der Düse einer Patrone vorzugsweise mit Hilfe einer eine Mikrodosierpumpe auf der Basis piezoelektronischer oder thermischer Aktoren erfolgen.

Der Produktbehälter der erfindungsgemäßen Vorrichtung kann in allen zweckdienlichen Formen ausgestaltet sein, vorzugsweise in der Form von Dosen, Flaschen, Patronen oder Tüten.

Bei einer besonders vorteilhaften Weiterentwicklung ist die erfindungsgemäße Vorrichtung in ein System zur Erzeugung einer virtuellen Realität integriert. Dazu kann die erfindungsgemäße Vorrichtung mobil oder stationär angeordnet sein. Weiterhin kann der erzeugte Duft frei in den Raum abgegeben werden oder in ein Leitungssystem für eine Luftzufuhr oder ein Beatmungssystem zu einem oder mehreren Nutzern der Virtuellen Realität geleitet werden.

Von Vorteil ist es ferner, wenn der nichtflüchtige Schreib-Lese-Speicher Daten zur eindeutigen Identifizierung des Produktbehälters enthält.

Ein weiterer Vorteil ergibt sich, wenn am Produktbehälter und/oder vor oder hinter der Austrag-Einheit ein Mittel zum vollständigen und/oder teilweisen Verschließen des Produktbehälters angeordnet ist. Dabei kann das Mittel zum vollständigen und/oder teilweisen Verschließen des Produktbehälters vorzugsweise ein regelbares Ventil sein.

Ferner ist es von Vorteil, wenn der Produktbehälter und/oder das Gehäuse einen oder mehrere Sensoren aufweisen, die die Menge an aromatischer Flüssigkeit, aromatischem Feststoff, aromatischem Gel und/oder mit einer aromatischen Flüssigkeit oder einem aromatischen Gel getränkten Feststoff im Produktbehälter bestimmen oder messen können.

Weiterhin ist es von Vorteil, wenn die am Gehäuse angeordnete Datenverarbeitungseinheit mit einem Computersystem verbunden ist.

Die Energieversorgung für das Mittel zum vollständigen und/oder teilweisen Verschließen des Produktbehälters und/oder für die Austrag-Einheit kann gegebenenfalls entweder über eine externe Energie-Quelle oder eine an der Vorrichtung selbst angeordnete Energie-Quelle erfolgen. Weiterhin kann die Energieversorgung auch direkt über eine der Datenverarbeitungseinheiten erfolgen.

Weiterhin ist im Sinne dieser Erfindung ein Verfahren zur Abgabe von Düften mit einer Vorrichtung nach einer der oben beschriebenen Ausführungsvarianten, wobei dabei ein Datenaustausch zwischen dem am Produktbehälter unlösbar angebrachten, nichtflüchtigen Schreib-Lese-Speicher der Datenverarbeitungseinheit und der am Gehäuse angeordneten Datenverarbeitungseinheit erfolgt, wobei Informationen zum jeweils im Gehäuse befindlichen Produktbehälter aus dem nichtflüchtigen Schreib-Lese-Speicher ausgelesen werden.

Bei einer besonders vorteilhaften Ausgestaltung des erfindungsgemäßen Verfahrens erfolgt ein gegenseitiger Datenaustausch zwischen der am Produktbehälter unlösbar angebrachten Datenverarbeitungseinheit und der am Gehäuse angeordneten Datenverarbeitungseinheit über eine Schnittstelle.

Ferner ist es von Vorteil, wenn die aromatische Flüssigkeit, der aromatischer Feststoff, das aromatisches Gel und/oder der mit einer aromatischen Flüssigkeit oder einem aromatischen Gel getränkte Feststoff mit Hilfe der Austrag-Einheit verdunstet, sublimiert und/oder zerstäubt werden.

Ferner ist es von Vorteil, wenn die Steuerung des Mittels zum vollständigen und/oder teilweisen Verschließen des Produktbehälters gegenüber der Austrag-Einheit durch eine Datenverarbeitungseinheit, vorzugsweise durch die Datenverarbeitungseinheit am Produktbehälter, erfolgt.

Ferner ergibt sich ein Vorteil daraus, dass beim Auswechseln des Produktbehälters, die darauf unlösbar befestigte Datenverarbeitungseinheit mit ausgetauscht wird, denn somit kann es nicht zu versehentlichen Verwechselungen der Produktbehälter kommen. Es kann weiterhin von Vorteil sein, wenn mit dem Produktbehälter gleichzeitig auch das Mittel zum vollständigen und/oder teilweisen Verschließen des Produktbehälters und/oder die Austrag-Einheit mit ausgetauscht werden.

Bei einer vorteilhaften Weiterentwicklung des erfindungsgemäßen Verfahrens wird bei einer geringen Menge an aromatische Flüssigkeit, an einer geringen Menge an aromatischen Feststoff und/oder an einer geringen Menge an einer aromatischen Flüssigkeit auf einem Feststoff am Produktbehälter und/oder am Gehäuse ein Warnsignal ausgegeben.

Weiterhin steuert eine Kontrolleinheit die Austrag-Einheit und/oder das Mittel zum vollständigen und/oder teilweisen Verschließen des Produktbehälters nur bei eindeutiger Identifizierung des Produktbehälters durch einen Daten-Abgleich mit den im nichtflüchtigen Schreib-Lese-Speicher gespeicherten Daten an.

Von Vorteil ist es ferner, wenn bei einer geringen Menge an aromatischer Flüssigkeit, aromatischem Feststoff, aromatischem Gel und/oder mit einer aromatischen Flüssigkeit oder einem aromatischen Gel getränktem Feststoff das Mittel zum vollständigen und/oder teilweisen Verschließen des Produktbehälters gegenüber der Austrag-Einheit in der geschlossenen Position blockiert wird.

Bei einer vorteilhaften Weiterentwicklung des Verfahrens sind im nichtflüchtigen Schreib-Lese-Speicher des Produktbehälters Eigenschaftsdaten der aromatischen Flüssigkeit, des aromatischen Feststoffs, des aromatisches Gels und/oder des mit einer aromatischen Flüssigkeit oder einem aromatischen Gel getränkten Feststoffs gespeichert.

Ferner ist es von Vorteil, wenn im nichtflüchtigen Schreib-Lese-Speicher mit Hilfe des mit der Kontrolleinheit oder mit dem nichtflüchtigen Schreib-Lese-Speicher verbundenen Timers die Gesamtzeit, die die aromatische Flüssigkeit, der aromatischer Feststoff, das aromatisches Gel und/oder der mit einer aromatischen Flüssigkeit oder einem aromatischen Gel getränkte Feststoff der Begasung ausgesetzt war, gespeichert wird. Für diese Funktion ist es wichtig, dass es sich bei dem nichtflüchtigen Speicher um einen Speicherbaustein mit Schreibfunktion handelt. Somit ergibt sich die vorteilhafte Möglichkeit, dass bei Übereinstimmung der im nichtflüchtigen Schreib-Lese-Speicher für den jeweiligen Produktbehälter gespeicherten maximalen Zeitdauer mit der im nichtflüchtigen Schreib-Lese-Speicher gespeicherten tatsächlichen Gesamtdauer der Begasung, die Austrag-Einheit und/oder das Mittel zum vollständigen und/oder teilweisen Verschließen des Produktbehälters in der geschlossenen Position blockiert werden.

In Abhängigkeit von der Ausgestaltung der Austrags-Einheit kann es auch von Vorteil sein, wenn im nichtflüchtigen Schreib-Lese-Speicher des Produktbehälters die tatsächlich bereits erfolgte Gesamtanzahl der Öffnungs- und/oder Verschlussvorgänge der Austrag-Einheit gespeichert wird. Daraus ergibt sich die vorteilhafte Möglichkeit, dass bei Übereinstimmung einer im nichtflüchtigen Schreib-Lese-Speicher gespeicherten Anzahl an maximalen Öffnungs- und/oder Verschlussvorgängen der Austrag-Einheit mit der Gesamtanzahl der tatsächlich erfolgten Öffnungs- und/oder Verschlussvorgänge ein Warnsignal mit Hilfe des akustischen und/oder visuellen Signalgebers abgegeben wird und das Mittel zum vollständigen und/oder teilweisen Verschließen des Produktbehälters in der geschlossenen Position blockiert wird.

Bei einer besonders vorteilhaften Ausführung des erfindungsgemäßen Verfahrens stimulieren die erzeugten Aerosole oder Gase den Geruchssinn eines oder mehrerer Menschen in einer virtuellen Realität.

Ferner ist es von Vorteil, wenn das Mittel zum vollständigen und/oder teilweisen Verschließen des Produktbehälters und/oder die Austrag-Einheit über ein Computersystem gesteuert werden. Über das Computersystem kann somit eine an eine virtuelle Realität angepasste Steuerung der Dufterzeugung erfolgen.

Nachfolgend wird die erfindungsgemäße Vorrichtung bzw. das erfindungsgemäße Verfahren am Beispiel einer Ausführungsvariante mit Bezugnahme auf die Zeichnungen näher erläutert. Dabei wird in der
- Fig. 1: eine Ausführungsvariante der erfindungsgemäßen Vorrichtung in Schnittansicht und in der
- Fig. 2: ein schematisches Schaltdiagramm der Datenverarbeitungseinheiten
dargestellt.

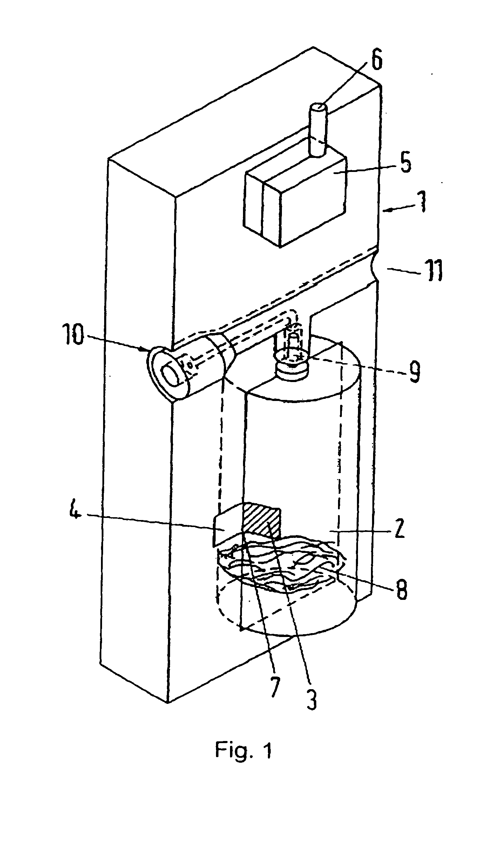

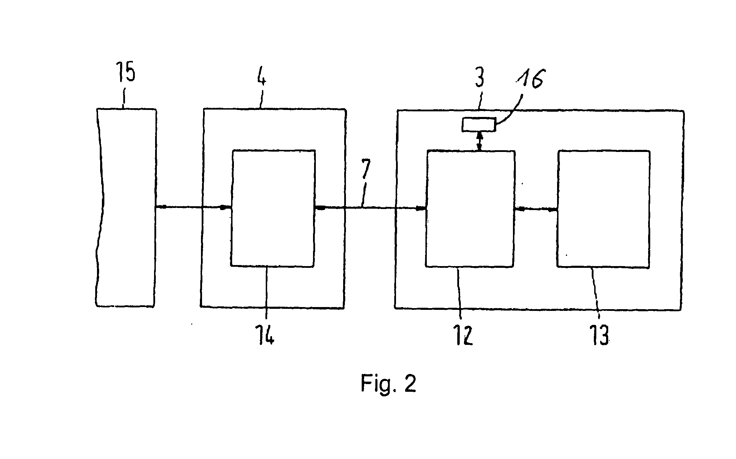

Gemäß der Fig. 1 und Fig. 2 besteht eine Ausführungsvariante der erfindungsgemäßen Vorrichtung zum Erzeugen von Düften aus einem Gehäuse 1, einem mit einem aromatischen Gel 8 befüllten Produktbehälter 2, einem Auslass 9 und einem Ventil 10, wobei der Duftstoff aus dem Gel 8 im Produktbehälter 2 verdunstet und bei offener Position des Auslasses 9 und geöffnetem Ventil 10 in die Leitung 11 eingeleitet wird. Dabei kann über die Leitung 11 einströmende Luft teilweise oder komplett in den in den Produktbehälter 2 geleitet werden und anschließend diesen über den Auslass 9 wieder verlassen. Eine Datenverarbeitungseinheit 4 mit einem Speicherelement 14 ist am Gehäuse 1 angebracht. Eine weitere Datenverarbeitungseinheit 3 ist unlösbar am Produktbehälter 2 befestigt, wobei beide Datenverarbeitungseinheiten 3, 4 über eine Schnittstelle 7 miteinander verbunden sind. Die Pfeile symbolisieren Datenübertragungen über jeweils eine oder mehrere Leitungen. Die Datenverarbeitungseinheit 3 enthält eine Kontrolleinheit 12, einen Timer 16 und einen nichtflüchtigen Speicher 13. Die Datenverarbeitungseinheit 4 ist mit einem Computersystem 15 verbunden. Der Auslass 9 und das Ventil 10 werden von der Datenverarbeitungseinheit 3 über in der Zeichnung nicht dargestellte Leitungen angesteuert. Der Produktbehälter 2 kann über eine in der Zeichnung nicht dargestellte Öffnung im Gehäuse 1 ausgetauscht werden. Am Gehäuse 1 sind weiterhin ein akustischer Signalgeber 5 und ein visueller Signalgeber 6 angeordnet, welche über in der Zeichnung nicht dargestellte Leitungen von der Kontrolleinheit 12 angesteuert werden.

### Bezugszeichenliste:

- 1: Gehäuse
- 2: Produktbehälter
- 3: Datenverarbeitungseinheit, am Produktbehälter
- 4: Datenverarbeitungseinheit, am Gehäuse
- 5: akustischer Signalgeber
- 6: visueller Signalgeber
- 7: Schnittstelle
- 8: aromatische Flüssigkeit, aromatischer Feststoff, aromatisches Gel und/oder ein mit einer aromatischen Flüssigkeit oder einem aromatischen Gel getränkter Feststoff
- 9: Austrag-Einheit
- 10: Mittel zum vollständigen und/oder teilweisen Verschließen des Produktbehälters, Auslass
- 11: Leitung
- 12: Kontrolleinheit
- 13: nichtflüchtiger Schreib-Lese-Speicher
- 14: Speicher-Element
- 15: Computersystem
- 16: Timer

## Patentansprüche

1. Vorrichtung zur Abgabe von Düften, wobei die Vorrichtung mindestens
- ein Gehäuse (1) mit einer elektronischen Datenverarbeitungseinheit (4),
- eine in oder an dem Gehäuse (1) befestigte Austrag-Einheit (9) für die Abgabe von aerosol- oder gasförmigen Duftstoffen,
- einen in oder an dem Gehäuse (1) austauschbar befestigten mit Duftstoffen bestückten Produktbehälter (2) aufweist, wobei der Produktbehälter (2) über eine weitere, unlösbar am Produktbehälter (2) befestigten, Datenverarbeitungseinheit (3) mit mindestens einem nichtflüchtigen Schreib-Lese-Speicher (13) verfügt, **dadurch gekennzeichnet, daß**
- die Datenverarbeitungseinheit (3) eine mit dem nichtflüchtigen Schreib-Lese-Speicher (13) verbundene Kontrolleinheit (12) zur Steuerung der Austrag-Einheit (9) enthält.

2. Vorrichtung nach Anspruch 1, **dadurch gekennzeichnet, dass** die Duftstoffe im Produktbehälter (2) eine aromatische Flüssigkeit, ein aromatischer Feststoff, ein aromatisches Gel und/oder ein mit einer aromatischen Flüssigkeit oder einem aromatischen Gel getränkter Feststoff (8) ist.

3. Vorrichtung nach Anspruch 1 oder 2, **dadurch gekennzeichnet, dass** die Datenverarbeitungseinheit (3) einen mit dem nichtflüchtigen Schreib-Lese-Speicher (13) oder der Kontrolleinheit verbundenen Timer (16) enthält.

4. Vorrichtung nach einem der Ansprüche 1 bis 3, **dadurch gekennzeichnet, dass** der nichtflüchtige Schreib-Lese-Speicher (13) ein elektronischer Speicherbaustein vom Typ eines EPROMs, EEPROMs oder Flash-Speichers ist.

5. Vorrichtung nach einem der Ansprüche 1 bis 4, **dadurch gekennzeichnet, dass** der Produktbehälter (2) mehrere voneinander abgetrennte und von der Kontrolleinheit (12) separat ansteuerbare Kammern für die Speicherung verschiedener Duftstoffe besitzt.

6. Vorrichtung nach einem der Ansprüche 1 bis 5, **dadurch gekennzeichnet, dass** die Vorrichtung in ein System zur Erzeugung einer virtuellen Realität integriert ist.

7. Vorrichtung nach einem der Ansprüche 1 bis 6, **dadurch gekennzeichnet, dass** der nichtflüchtige Schreib-Lese-Speicher (13) Daten zur eindeutigen Identifizierung des Produktbehälters (2) enthält.

8. Vorrichtung nach einem der Ansprüche 1 bis 7, **dadurch gekennzeichnet, dass** am Produktbehälter (2) und/oder vor oder hinter der Austrag-Einheit (9) ein Mittel (10) zum vollständigen und/oder teilweisen Verschließen des Produktbehälters (2) angeordnet ist.

9. Vorrichtung nach einem der Ansprüche 1 bis 8, **dadurch gekennzeichnet, dass** das Mittel (10) zum vollständigen und/oder teilweisen Verschließen des Produktbehälters (2) ein regelbares Ventil ist.

10. Vorrichtung nach einem der Ansprüche 1 bis 9, **dadurch gekennzeichnet, dass** der Produktbehälter (2) und/oder das Gehäuse (1) einen oder mehrere Sensoren aufweisen, die die Menge an aromatischer Flüssigkeit, aromatischem Feststoff, aromatischem Gel und/oder mit einer aromatischen Flüssigkeit oder einem aromatischen Gel getränkten Feststoff (8) im Produktbehälter (2) bestimmen.

11. Verfahren zur Abgabe von Düften mit einer Vorrichtung umfassend ein Gehäuse (1) mit einer elektronischen Datenverarbeitungseinheit (4), eine in oder an dem Gehäuse (1) befestigten Austrag-Einheit (9) für die Abgabe von aerosol- oder gasförmigen Duftstoffen sowie einen in oder an dem Gehäuse (1) austauschbar befestigten und mit Duftstoffen bestückten Produktbehälter (2), wobei der Produktbehälter (2) über eine weitere, unlösbar am Produktbehälter (2) befestigte, Datenverarbeitungseinheit (3) mit mindestens einem nichtflüchtigen Schreib-Lese-Speicher (13) verfügt, **dadurch gekennzeichnet, dass** ein Datenaustausch zwischen dem am Produktbehälter (2) unlösbar angebrachten, nichtflüchtigen Schreib-Lese-Speicher (13) der Datenverarbeitungseinheit (3) und der am Gehäuse (1) angeordneten Datcnvcrarbeirungseinhcit (4) erfolgt, wobei Informationen zum jeweils im Gehäuse (I) befindlichen Produktbehälter (2) aus dem nichtflüchtigen Schreib-Lese-Speicher (13) ausgelesen werden und eine mit dem nichtflüchtigen Schreib-Lese-Speicher (13) verbundene Kontrolleinheit (12) die Austrag-Einheit (9) und/oder das Mittel (10) zum vollständigen und/oder teilweisen Verschließen des Produktbehälters (2) nur bei eindeutiger Identifizierung des Produktbehällers (2) durch einen Daten-Abgleich mit den im nichtflüchtigen Schreib-Lcse-Speicher (13) gespeicherten Daten ansteuert.

12. Verfahren nach Anspruch 11, **dadurch gekennzeichnet, dass** zwischen der Datenverarbeitungseinheit (3) und der Datenverarbeitungseirtheat (4) über eine Schnittstelle (7) ein gegenseitiger Datenaustausch erfolgt.

13. Verfahren nach Anspruch 11 oder 12, **dadurch gekennzeichnet, dass** die aromatische Flüssigkeit, der aromatischer Feststoff, das aromatisches Gel und/oder der mit einer aromatischen Flüssigkeit oder einem aromatischen Gel getränkze Feststoff (8) mit Hilfe der Austrag-Einheit (9) verdunstet, sublimiert und/oder zerstäubt werden.

14. Verfahren nach einem der Ansprüche 11 bis 13, **dadurch gekennzeichnet, dass** die erzeugten Aerosole oder Gase den Geruchssinn eines oder mehrerer Menschen in einer virtuellen Realität stimulierten.

15. Verfahren nach einem der Ansprüche 11 bis 14, **dadurch gekennzeichnet, dass** die Steuerung des Mittels (10) zum vollständigen und/oder teilweisen Verschließen des Produktbchälters (2) durch die Datenverarbeitungseinheit (3) arm Produktbehälter (2), erfolgt.

## Claims

1. A device for releasing fragrances, wherein the device has at least
• a housing (1) with an electronic data processing unit (4),
• a dispensing unit (9) for the release of aerosol or gaseous fragrances which is attached in or on the housing (1),
• a product container (2) loaded with fragrances which is replaceably attached in or on the housing (1), wherein said product container (2) has another data processing unit (3) with at least one non-volatile read-write memory (13), said data processing unit being inseparably attached to the product container (2),
**characterized in that**
the data processing unit (3) includes a control unit (12) to control the dispensing unit (9), said control unit being connected to the non-volatile read-write memory (13).

2. The device of claim 1, **characterized in that**
the fragrance in the product container (2) is an aromatic fluid, an aromatic solid, an aromatic gel and/or a solid soaked in an aromatic fluid or an aromatic gel (8).

3. The device of claim 1 or 2, **characterized in that**
the data processing unit (3) includes a timer (16), said timer being connected to the non-volatile read-write memory (13) or the control unit.

4. The device of any one of claims 1 to 3, **characterized in that**
the non-volatile read-write memory (13) is an electronic memory module of the EPROM, EEPROM or flash memory type.

5. The device of any one of claims 1 to 4, **characterized in that** the product container (2) has several mutually separated chambers for the storage of different fragrances, said chambers being separately controllable by the control unit.

6. The device of any one of claims 1 to 5, **characterized in that**
the device is integrated into a system for creating a virtual reality.

7. The device of any one of claims 1 to 6, **characterized in that**
the non-volatile read-write memory (13) includes data for the unambiguous identification of the product container (2).

8. The device of any one of claims 1 to 7, **characterized in that**
a means (10) for partially and/or completely locking the product container (2) is arranged on the product container (2) and/or in front of or behind the dispensing unit (9).

9. The device of any one of claims 1 to 8, **characterized in that**
the means (10) for partially and/or completely locking the product container (2) is an adjustable valve.

10. The device of any one of claims 1 to 9, **characterized in that**
the product container (2) and/or the housing (1) have one or several sensors which determine the amount of aromatic fluid, aromatic solid, aromatic gel and/or solid soaked in an aromatic fluid or an aromatic gel (8) in the product container (2).

11. A method for releasing fragrances with a device comprising
a housing (1) with an electronic data processing unit (4), a dispensing unit (9) for the release of aerosol or gaseous fragrances which is attached in or on the housing (1) and a product container (2) loaded with fragrances which is replaceably attached in or on the housing (1), wherein said product container (2) has another data processing unit (3) with at least one non-volatile read-write memory (13), said data processing unit being inseparably attached to the product container (2),
**characterized in that**
an exchange of data takes place between the non-volatile read-write memory (13) of the data processing unit (3) inseparably attached to the product container (2) and the data processing unit (4) arranged on the housing (1), wherein information about the product container (2) present in the housing (1) is read out from the non-volatile read-write memory (13), and a control unit (12) connected to the non-volatile read-write memory (13) only controls the dispensing unit (9) and/or the means (10) for partially and/or completely locking the product container (2) if the product container (2) is unambiguously identified through data matching with the data stored in the non-volatile read-write memory (13).

12. The method of claim 11, **characterized in that**
a mutual exchange of data takes place between the data processing unit (3) and the data processing unit (4) via an interface (7).

13. The method of claim 11 or 12, **characterized in that**
the aromatic fluid, the aromatic solid, the aromatic gel and/or the solid soaked in an aromatic fluid or an aromatic gel (8) is evaporated, sublimated and/or atomised using the dispensing unit (9).

14. The method of any one of claims 11 to 13, **characterized in that**
the generated aerosols or gases stimulate the sense of smell of one or several people in a virtual reality.

15. The method of any one of claims 11 to 14, **characterized in that**
the means (10) for partially and/or completely locking the product container (2) is controlled by the data processing unit (3) at the product container (2).

## Revendications

1. Dispositif pour diffuser des senteurs, le dispositif présentant au moins
- un boîtier (1) avec une unité de traitement électronique de données (4),
- une unité de sortie (9) fixée dans ou sur le boîtier (1) pour la diffusion de matières odoriférantes sous forme d'aérosols ou de gaz,
- un réservoir de produit (2) muni de matières odoriférantes et fixé de façon remplaçable dans ou sur le boîtier (1), le réservoir de produit (2) disposant d'une autre unité de traitement de données (3) fixée de façon non détachable sur le réservoir de produit (2), avec au moins une mémoire écriture-lecture (13) non volatile, **caractérisé en ce que**
- l'unité de traitement de données (3) contient une unité de contrôle (12) raccordée à la mémoire écriture-lecture (13) non volatile et destinée à commander l'unité de sortie (9).

2. Dispositif selon la revendication 1, **caractérisé en ce que** les matières odoriférantes dans le réservoir de produit (2) sont un liquide aromatique, une matière solide aromatique, un gel aromatique et/ou une matière solide (8) humectée avec un liquide aromatique ou un gel aromatique.

3. Dispositif selon la revendication 1 ou 2, **caractérisé en ce que** l'unité de traitement de données (3) contient un minuteur (16) raccordé à la mémoire écriture-lecture (13) non volatile ou à l'unité de contrôle (12).

4. Dispositif selon une des revendications 1 à 3, **caractérisé en ce que** la mémoire écriture-lecture (13) non volatile est un module de mémoire électronique du type mémoire EPROM, EEPROM ou flash.

5. Dispositif selon une des revendications 1 à 4, **caractérisé en ce que**, pour le stockage de différentes matières odoriférantes,le réservoir de produit (2) possède plusieurs chambres séparées les unes des autres et pouvant être pilotées séparément par l'unité de contrôle (12).

6. Dispositif selon une des revendications 1 à 5, **caractérisé en ce que** le dispositif est intégré dans un système de production d'une réalité virtuelle.

7. Dispositif selon une des revendications 1 à 6, **caractérisé en ce que** la mémoire écriture-lecture (13) non volatile contient des données pour l'identification explicite du réservoir de produit (2).

8. Dispositif selon une des revendications 1 à 7, **caractérisé en ce que**, sur le réservoir de produit (2) et/ou devant ou derrière l'unité de sortie (9), il est disposé un moyen (10) pour la fermeture complète et/ou partielle du réservoir de produit (2).

9. Dispositif selon une des revendications 1 à 8, **caractérisé en ce que** le moyen (10) pour la fermeture complète et/ou partielle du réservoir de produit (2) est un robinet réglable.

10. Dispositif selon une des revendications 1 à 9, **caractérisé en ce que** le réservoir de produit (2) et/ou le boîtier (1) présentent un ou plusieurs capteurs qui déterminent dans le réservoir de produit (2) la quantité de liquide aromatique, de matière solide aromatique, de gel aromatique ou de matière solide (8) humectée avec un liquide aromatique ou un gel aromatique.

11. Procédé pour diffuser des senteurs avec un dispositif comprenant un boîtier (1) avec une unité de traitement électronique de données (4), une unité de sortie (9) fixée dans ou sur le boîtier (1) pour la diffusion de matières odoriférantes sous forme d'aérosols ou de gaz, ainsi qu'un réservoir de produit (2) fixé de façon remplaçable dans ou sur le boîtier (1) et muni de matières odoriférantes, le réservoir de produit (2) disposant d'une autre unité de traitement de données (3) fixée de façon non détachable sur le réservoir de produit (2), avec au moins une mémoire écriture-lecture (13) non volatile, **caractérisé en ce qu'**un échange de données s'effectue entre la mémoire écriture-lecture (13) non volatile de l'unité de traitement de données (3) mise en place de façon non détachable sur le réservoir de produit (2) et l'unité de traitement de données (4) disposée sur le boîtier (1), des informations portant sur le réservoir de produit (2) situé respectivement dans le boîtier (1) étant extraites de la mémoire écriture-lecture (13) non volatile,et une unité de contrôle (12) raccordée à la mémoire écriture-lecture (13) non volatile ne pilotant l'unité de sortie (9) et/ou le moyen (10) pour la fermeture complète et/ou partielle du réservoir de produit (2) qu'en cas d'identification explicite du réservoir de produit (2) par une comparaison de données avec les données stockées dans la mémoire écriture-lecture (13) non volatile.

12. Procédé selon la revendication 11, **caractérisé en ce que**, entre l'unité de traitement de données (3) et l'unité de traitement de données (4), un échange de donnée réciproque s'effectue par le biais d'une interface (7).

13. Procédé selon la revendication 11 ou 12, **caractérisé en ce que** le liquide aromatique, la matière solide aromatique, le gel aromatique et/ou la matière solide (8) humectée avec un liquide aromatique ou un gel aromatique sont vaporisés, sublimés et/ou pulvérisés à l'aide de l'unité de sortie (9).

14. Procédé selon une des revendications 11 à 13, **caractérisé en ce que** les aérosols ou les gaz produits stimulent l'odorat d'une ou de plusieurs personnes dans une réalité virtuelle.

15. Procédé selon une des revendications 11 à 14, **caractérisé en ce que** la commande du moyen (10) pour la fermeture complète et/ou partielle du réservoir de produit (2) est effectuée par l'unité de traitement de données (3) sur le réservoir de produit (2).
